Europäisches Patentamt

European Patent Office

Office européen des brevets

(19)

(11) **EP 1 127 275 B1**

(12) **FASCICULE DE BREVET EUROPEEN**

(45) Date de publication et mention
de la délivrance du brevet:
**19.02.2003 Bulletin 2003/08**

(21) Numéro de dépôt: **99954069.3**

(22) Date de dépôt: **05.11.1999**

(51) Int Cl.⁷: $G01N\ 33/24$, $G01N\ 3/34$, $G01C\ 7/04$

(86) Numéro de dépôt international:
**PCT/FR99/02709**

(87) Numéro de publication internationale:
**WO 00/028320 (18.05.2000 Gazette 2000/20)**

(54) **PROCEDE ET APPAREIL POUR MESURER LA PORTANCE D'UNE PLATEFORME**

VERFAHREN UND VORRICHTUNG ZUR MESSUNG DER TRAGFÄHIGKEIT EINES UNTERBAUS

METHOD AND APPARATUS FOR MEASURING THE LOAD BEARING CAPACITY OF A PLATFORM

(84) Etats contractants désignés:
**AT BE CH CY DE DK ES FI FR GB GR IE IT LI LU
MC NL PT SE**

(30) Priorité: **05.11.1998 FR 9813922**

(43) Date de publication de la demande:
**29.08.2001 Bulletin 2001/35**

(73) Titulaire: **LABORATOIRE CENTRAL DES PONTS
ET CHAUSSEES
75015 Paris (FR)**

(72) Inventeurs:
• **QUIBEL, Alain
F-76800 Saint Etienne du Rouvray (FR)**
• **FROUMENTIN, Michel
F-76230 Bois Guillaume (FR)**

• **MARIGNIER, Jacques
F-76240 Mesnil-Esnard (FR)**
• **LEROY, Maurice
F-76300 Sotteville les Rouen (FR)**
• **MOREL, Guy
F-76120 Caudebec les Elbeuf (FR)**

(74) Mandataire: **Le Bras, Hervé et al
Cabinet Beau de Loménie,
158, rue de l'Université
75340 Paris Cedex 07 (FR)**

(56) Documents cités:
**EP-A- 0 589 772        EP-A- 0 646 771
FR-A- 2 076 423        FR-A- 2 334 785
US-A- 3 427 877**

EP 1 127 275 B1

**Description**

L'invention concerne un procédé pour mesurer en continu la raideur ou le module élastique d'une plateforme d'infrastructure de transport et sols industriels en cours de travaux ou en exploitation, et un appareil pour la mise en oeuvre du procédé.

Le procédé s'applique à des couches de forme, des arases de terrassements, ou tout autre plateforme dont le module espéré est compris entre 30 MPa et 300 MPa et qui présente une géométrie satisfaisante pour la circulation de l'appareil.

Le contrôle de la portance des arases de terrassements et des couches de forme est actuellement réalisé par l'un des appareils suivants :

- le camion d'essai à la plaque,
- la dynaplaque,
- le déflectographe.

Les deux premiers appareils réalisent une mesure ponctuelle. Pour avoir une vue d'ensemble de l'homogénéité de la portance et du respect des exigences requises, il convient d'effectuer les contrôles selon un maillage préétabli. L'essai à la plaque statique convient bien pour des modules jusqu'à 200 MPa, mais est relativement long.

La dynaplaque est un appareil à choc dans lequel la déformabilité de la plateforme auscultée est appréciée par le coefficient de restitution qui représente le rapport entre les hauteurs de chute et de rebond d'une masse tombant sur une série de ressorts disposés sur une plaque de 600 mm de diamètre posée sur le sol. L'essai à la dynaplaque est plus rapide que l'essai à la plaque statique. Sa sensibilité est maximale autour de 50 MPa, mais devient mauvaise au-delà de 100 MPa du fait de la forme de la courbe d'étalonnage entre le module élastique et le coefficient de restitution, compte tenu du choix des caractéristiques de l'appareil.

Le déflectographe délivre l'information sur la déflexion, engendrée par une charge roulante, et qui est représentable de façon quasi continue dans le profil en long. Par sa conception, il n'intervient que sur les chantiers d'accès aisé, et où l'uni et la déflexion ont normalement des caractéristiques favorables, tels que sur les sols traités et de faible granulométrie.

Il existe également une version améliorée de dynaplaque dont les caractéristiques dimensionnelles sont inchangées, mais dont le principe de mesure est différent. Dans cette version améliorée, la plaque de charge comporte une cellule de charge permettant d'accéder à l'acquisition de la force F transmise au sol en fonction du temps lors de l'impact ainsi qu'un capteur de déplacement vertical durant le choc. La combinaison de ces deux informations donne la courbe effort/déformation et permet d'accéder à la valeur du module. Cette méthode exploite la branche montante de la courbe force/déflexion obtenue pendant un cycle d'impact, et elle fournit une mesure plus sensible que celle obtenue par la dynaplaque traditionnelle, et ce jusqu'à un niveau de module de l'ordre de 200 MPa. Mais la mesure reste ponctuelle.

FR-A-2 334 785 et EP-A-0 646 771 décrivent des procédés pour mesurer en continu la raideur d'une plateforme, dans lesquels

- on fait rouler sur la plateforme une roue de masse Mr suspendue sous un châssis de masse Mc, et
- on soumet la roue à des vibrations verticales au moyen d'un balourd tournant autour de l'axe de la roue et soumis à une force centrifuge Fc.

Le procédé selon l'invention est caractérisé par les étapes suivantes :

- on mesure en fonction du temps l'accélération verticale Gr de la roue et l'accélération verticale Gc du châssis au moyen de capteurs d'accélération montés sur la roue et sur le châssis,
- on mesure en fonction du temps la phase angulaire $\varphi$ entre la direction de la force centrifuge Fc du balourd et la verticale au moyen d'un capteur,
- on calcule en fonction du temps la composante verticale FTA de la force appliquée par la roue sur la plateforme,
- on calcule en fonction du temps la déformation d verticale subie par la plateforme à partir de la mesure de l'accélération verticale Gr de la roue,
- on établit pour chaque cycle de vibration la boucle de la composante verticale FTA par rapport à la déformation d,
- on calcule à chaque cycle de vibration la pente de la boucle dans la branche des composantes verticales ascendantes pour obtenir la raideur de la plateforme.

Les avantageuses dispositions suivantes sont en outre adoptées :

- on calcule la pente de la courbe dans la plage des composantes verticales ascendantes comprises entre 30% et 90% de la composante verticale maximale ;
- le module élastique est calculé en multipliant la valeur de la raideur par un coefficient d'étalonnage C déterminé par une expérimentation préalable ;
- la fréquence des vibrations de la roue est comprise entre 20 et 50 Hz;
- on fait avancer la roue à une vitesse voisine de 1m/s ;
- on mesure en continu la vitesse de déplacement de la roue ;
- on calcule la raideur moyenne de la plateforme par unité de parcours en calculant la moyenne des raideurs calculées au cours des cycles de vibrations correspondant à cette unité de parcours ;
- on affiche en continue les raideurs moyennes calculées en fonction de la distance sur un écran de visualisation.

L'invention concerne également un appareil pour mettre en oeuvre le procédé selon l'invention.

Cet appareil est caractérisé par le fait qu'il comporte

- une remorque tractable reposant sur des roues de roulement au sol,
- un châssis de masse Mc monté sur la remorque de manière à pouvoir pivoter autour d'un axe transversal,
- une roue de masse Mr équipée d'un balourd et suspendue sous le châssis,
- des moyens pour faire tourner le balourd,
- un premier capteur d'accélération monté sur le châssis,
- un deuxième capteur d'accélération monté sur la roue,
- un capteur d'angle du balourd,
- des moyens pour calculer en fonction du temps la composante de la force appliquée par la roue sur la plateforme,
- des moyens pour calculer en fonction du temps la déformation verticale subie par la plateforme,
- des moyens pour établir à chaque cycle de vibration la composante verticale par rapport à la déformation, et des moyens pour calculer la raideur de la plateforme à chaque cycle de vibration.

D'autres avantages et caractéristiques de l'invention ressortiront à la lecture de la description suivante faite à titre d'exemple et en référence aux dessins annexés dans lesquels :

- la figure 1 est une vue latérale de l'appareil selon l'invention en mode transport,
- la figure 2 est une vue arrière de l'appareil de la figure 1, partiellement en coupe,
- la figure 3 est une vue latérale de l'appareil selon l'invention en mode utilisation,
- la figure 4 est une vue arrière de l'appareil de la figure 3 en mode utilisation,
- la figure 5 est une représentation schématique de la roue et du châssis en mode utilisation,
- la figure 6 montre la courbe représentative de la force totale appliquée au cours d'une période ou d'une moyenne de 20 périodes,
- la figure 7 montre la courbe représentative de la déflexion au cours d'une période,
- la figure 8 montre la boucle force/déflexion, et
- la figure 9 montre l'architecture du système d'acquisition de données et de calcul.

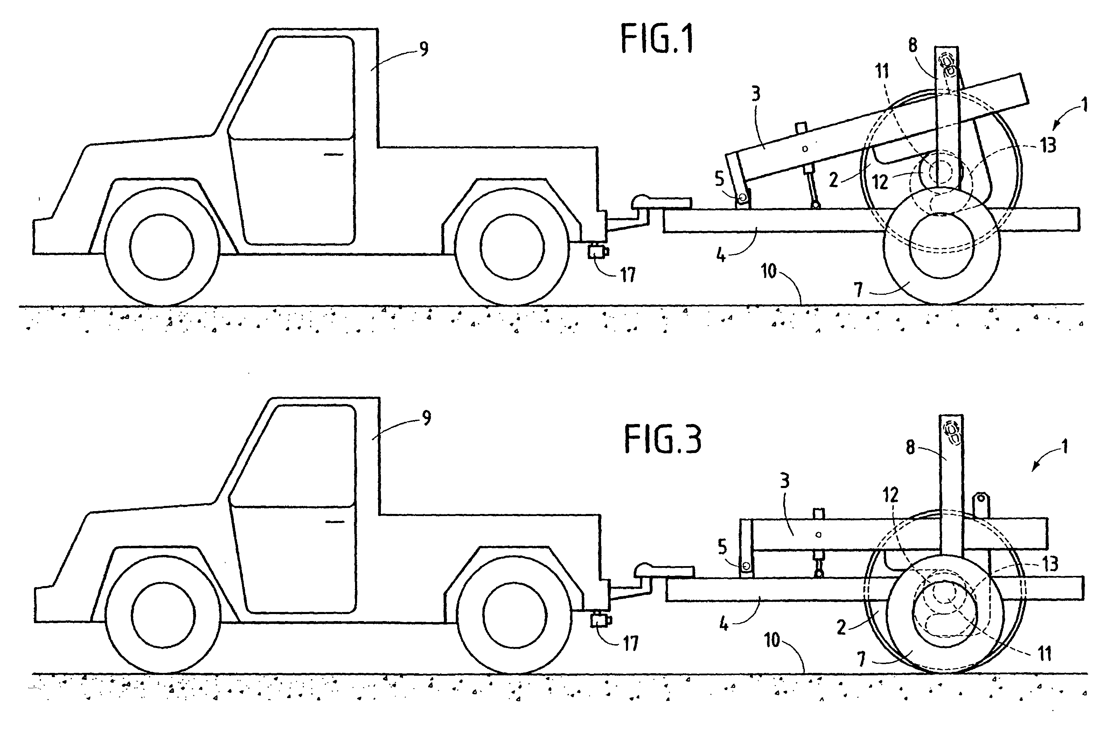

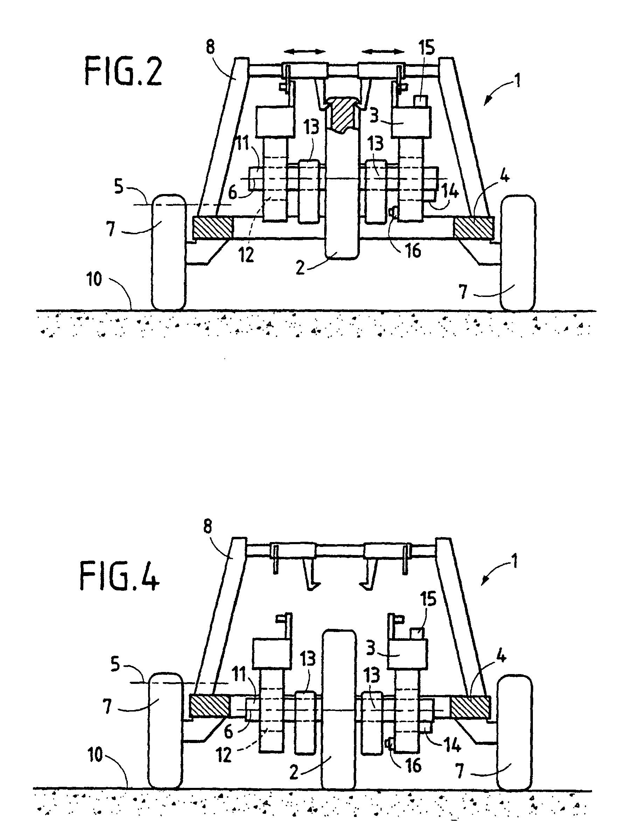

Les figures 1 à 4 montrent un appareil 1 destiné à mesurer en continu la raideur et le module élastique d'une plateforme d'infrastructure de transport et sols industriels en cours de travaux ou en exploitation. L'appareil comporte essentiellement une roue 2 de masse Mr suspendue sous un châssis 3 de masse Mc. Le châssis 3 est monté sur une remorque 4 de manière à pouvoir pivoter librement autour d'un axe horizontal et transversal 5 parallèle à l'axe 6 de la roue 2. La remorque 4 comporte deux roues 7 de roulement sur le sol et une potence 8 qui sert à maintenir la roue 2 et le châssis 3 dans une position relevée, ainsi que cela est montré sur les figures 1 et 2, lors du transport de l'appareil 1 d'un chantier vers un autre chantier. La remorque 4 est tractée par un véhicule 9 lors de son déplacement sur route et également lors de son utilisation sur un chantier.

Les figures 3 et 4 montrent l'appareil 1 au cours de son utilisation sur un chantier. Dans cette situation, la roue 2 et le châssis 3 sont désolidarisés de la potence 8. La roue 2 repose alors sur la plateforme 10 dont on veut mesurer la raideur et le module élastique, et roule librement lors de l'avancement de la remorque 4 tractée par le véhicule 9.

L'axe de pivotement 5 du châssis 3 est de préférence situé à l'avant de la remorque 4 et est éloigné de l'axe de rotation 6 de la roue 2, cette dernière étant disposée à l'arrière du châssis 3.

En position de travail de l'appareil 1, la remorque 4 a pour rôle de tracter le châssis 3 et la roue 2 et de maintenir la roue 2 dans un plan sensiblement vertical parallèle à la direction d'avancement du véhicule 9.

La roue 2 comporte un arbre horizontal 11 monté sur le châssis 3 au moyen de paliers 12 qui permettent des déplacements verticaux relatifs entre la roue 2 et le châssis 3.

L'arbre horizontal 11 de la roue 2 est équipé de balourds 13 synchronisés qui sont entraînés en rotation autour de l'axe 6 à une vitesse constante comprise entre 20 et 50 tours par seconde, au moyen d'un moteur de préférence hydraulique. L'énergie nécessaire pour entraîner ce moteur est fournie par le véhicule 9, afin que la masse de l'appareil 1 soit constante pendant la durée de la mesure.

Les balourds 13 sont soumis à une force centrifuge Fc fonction du moment d'exentricité des balourds 13 et de leur vitesse de rotation.

La roue 2 et le châssis 3 sont alors soumis à des vibrations verticales à une fréquence comprise entre 20 et 50 Hz selon la vitesse de rotation des balourds.

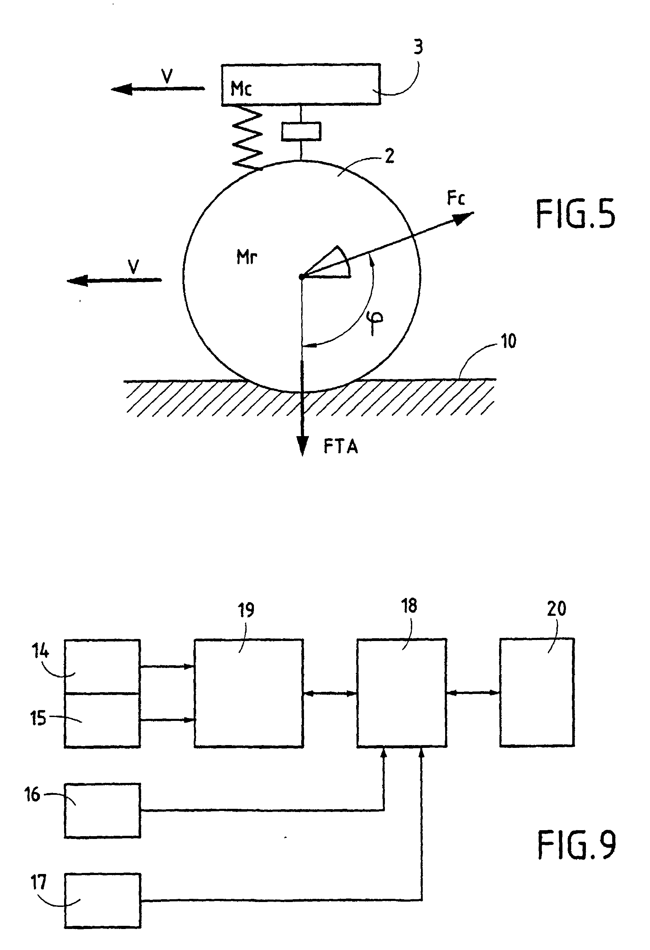

La figure 5 montre la représentation schématique de la composante verticale, appelée force totale appliquée FTA, des forces appliquées par la roue 2, le châssis 3 et les balourds 13 sur la plateforme 10.

Cette composante verticale FTA est la somme des forces suivantes :

- le poids de la roue 2 et du châssis 3, soit (Mr+Mc)x g, g étant l'attraction terrestre,
- la force d'inertie de la roue 2, soit Mr x Gr, Gr étant la composante verticale de l'accélération de la roue 2,
- la force d'inertie du châssis 3, soit Mc x Gc, Gc étant la composante verticale de l'accélération du châssis 3, et
- la composante verticale de la force centrifuge Fc des balourds 13, soit Fc x cos φ, φ étant le déphasage entre la direction de la force centrifuge Fc et la verticale.

On a donc l'équation :

$$FTA = (Mr + Mc) \times g + Mr \times Gr + Mc \times Gc + Fc \times \cos \varphi$$

Les masses Mr et Mc, sont des constantes mesurables et la force centrifuge Fc est constante puisque la vitesse de rotation des balourds est constante.

La mesure des accélérations Gr et Gc et la mesure de l'angle φ en fonction du temps permettent donc de calculer en fonction du temps la force FTA appliquée par la roue 2 sur la plateforme 10.

Selon la présente invention, la roue 2 est équipée d'un accéléromètre 14 du type piezo-électrique et le châssis 3 est également équipé d'un accéléromètre 15 du type piezo-électrique.

L'angle φ calculé en fonction du temps grâce à un capteur optique 16 qui délivre une impulsion à chaque passage bas des balourds 13.

La valeur fournie par l'accéléromètre 14 qui mesure l'accélération verticale de la roue 2 est utilisée pour calculer l'amplitude verticale de vibration par intégration double.

Cette intégration peut être réalisée après décomposition en série de fourrier du signal accélération Gr et intégration double des termes de la série.

L'amplitude verticale de vibration correspond à la déformation verticale d ou déflexion subie par la plateforme 10 au moment de l'impact.

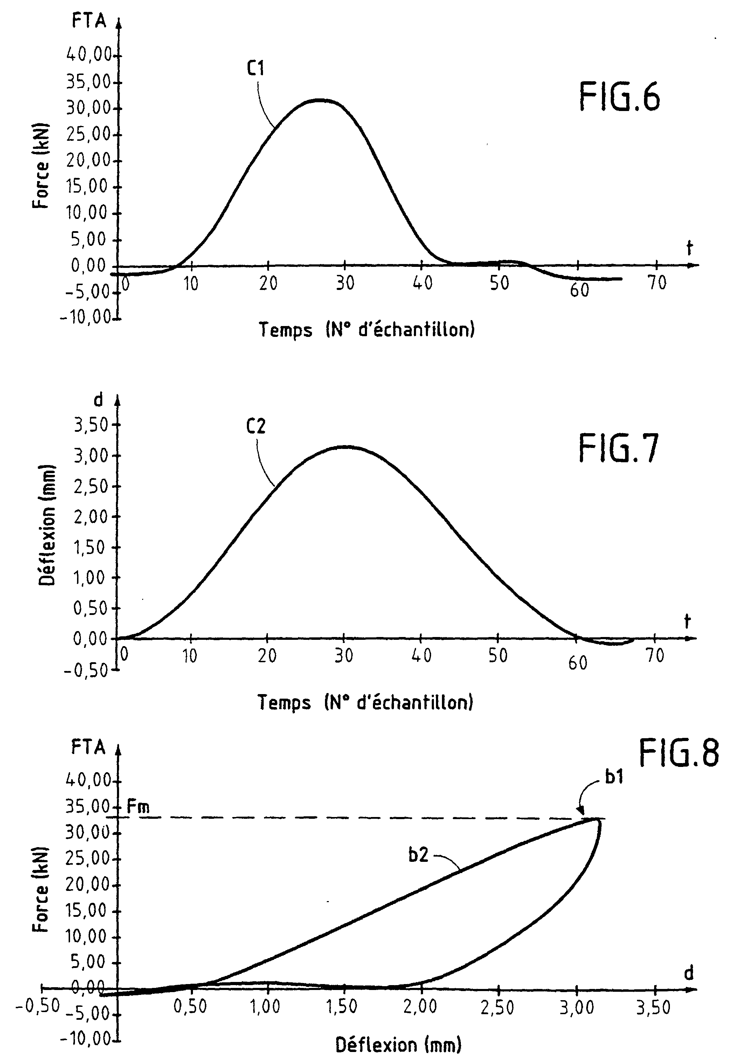

La figure 6 montre la courbe (C1) de variation de la force FTA en fonction du temps t pour un cycle de vibration, et la figure 7 montre la courbe (C2) de la variation de la déflexion d de la plateforme 10 en fonction du temps t pour le même cycle de variation. La figure 8 montre la bouche (b1) représentative de la variation de la force FTA en fonction de la déflexion d de la plateforme 12. Cette bouche (b1) comporte une branche supérieure (b2) qui correspond aux composantes verticales ascendantes.

Cette branche (b2) présente une portion sensiblement rectiligne dont la pente donne la mesure de la raideur de la plateforme 10.

De manière pratique, la pente est mesurée sur la portion de la branche supérieure (b2) comprise entre 30% et, 90% de la force maximale Fm mesurée.

Le véhicule 9 est en outre équipé à l'arrière d'un radar doppler 17 qui permet de mesurer la vitesse du véhicule 9 et par le fait même la distance parcourue par la roue 2.

Les accéléromètres 14 et 15 sont reliés à une unité centrale 18 portée par le véhicule 9 par l'intermédiaire d'une carte d'acquisition de données analogiques 19. Le capteur optique 16 et le radar doppler 17 sont également reliés à l'unité centrale 18 et cette dernière communique avec un micro-ordinateur 20 disposé au poste de conduite du véhicule 9.

La figure 9 représente l'architecture de l'ensemble, réalisé pour fonctionner en temps réel. par un système VME bâti autour d'un microprocesseur 68020 par exemple et d'un noyau temps réel P SOS.

Le noyau gère les tâches d'acquisition, de synchronisation, de traitement, de transfert et de dialogue avec le micro-ordinateur 20, ainsi que le contrôle de l'ensemble.

Le processus d'acquisition et d'échantillonnage est synchronisé sur la base de l'information des tours de balourds fournie par le capteur optique 16, qui déclenche une série de mesures, par échantillonnages couvrant 20 périodes successives. La production d'une période unique à partir de la moyenne de ces 20 périodes est réalisée en temps réel pendant la tâche d'acquisition. La tâche de traitement consiste à réaliser l'ensemble des calculs aboutissant à la valeur de la rigidité de la plateforme 10. Ensuite, le transfert des données utiles au micro-ordinateur 20 est réalisé. Ces données comportent le numéro d'enregistrement, la fréquence de vibration, la vitesse de translation, la distance cumulée, la rigidité calculée, la force totale appliquée maximale Fm et l'amplitude de la vibration.

Dès le début du traitement, une nouvelle séquence d'acquisition est lancée. Le nombre de 20 périodes résulte du souhait d'exprimer la portance par zones élémentaires de plus d'un mètre, et de la nécessité que le temps d'acquisition soit au moins égal à la sommation des temps de traitement et de transfert pour ne pas perdre l'information de l'acquisition.

A partir des signaux échantillonnés, le traitement consiste, par sommation des forces, à déterminer la force totale appliquée FTA qui est lissée par une décomposition de fourier de l'ordre 5, et à procéder à la double intégration de Gr par série de fourier à l'ordre 10 pour remonter à la valeur de la déflexion d.

Les deux valeurs FTA et d sont rassemblées dans un graphe (voir figure 8) d'où il ressort qu'au moyen d'un paramètrage convenable des bornes (exemple : borne inférieure 30% de Fm et borne supérieure 90% de Fm), le traitement par régression de la partie utile (b2) de la courbe (b1) fournit la valeur de la rigidité de la plateforme affectable à la zone auscultée pendant les 20 périodes. Il est à noter que 20 périodes de vibrations à une fréquence de 35 Hz correspond à une durée de 0,57 secondes, pendant laquelle l'avancement de l'appareil 1 est de l'ordre de 0,50 m à la vitesse de 1m/s. Il est également à noter que la fréquence d'échantillonnage de 2 KHz permet d'obtenir 57 points par période à 35 Hz.

L'affichage de la rigidité calculée en fonction de la distance parcourue sur un écran raccordé au microprocesseur 20 permet à l'opérateur ou conducteur du véhicule 9 d'avoir une visualisation de la courbe de la rigidité en fonction de la distance parcourue, sous la forme d'un graphe.

A titre informatif, la masse Mr de la roue 2 est de 600 Kg, la masse Mc du châssis 3 est de 400 Kg , le diamètre de la roue 2 est de 1m et la largeur de la roue 2 est de 0,10 m.

Un coefficient d'étallonage C déterminé à partir d'une expérimentation préalable de l'appareil 1 par rapport à des méthodes en vigueur pour mesurer le module élastique des plateformes, permet d'obtenir la mesure du module élastique de la plateforme 10 auscultée à partir de la raideur mesurée par l'appareil 1. Le coefficient d'étalonnage C est une constante de l'appareil 1 et correspond à un choix fixé de caractéristiques de conception de l'appareil, par exemple son amplitude de vibration, et de fonctionnement, par exemple la vitesse.

Au point de vue pratique, les balourds 13 peuvent prendre deux valeurs, suivant le sens de rotation appliqué.

L'appareil 1 est apte à fonctionner avec une petite amplitude, dans ce cas le sens de rotation des balourds 13 est opposé au sens de rotation de la roue 2 lors de l'avancement de l'appareil 1. Ce réglage correspond au cas usuel d'auscultation des plateformes 10 entre 30 et 300 MPa.

Il est également apte à fonctionner avec une grande amplitude, dans ce cas le sens de rotation des balourds 13 est le même que celui de la roue 2. Ce réglage est uniquement valable avec des plateformes 10 ayant des modules inférieurs à 80 MPa, et permet d'accroître la précision de la mesure dans la gamme 30-60 MPa notamment. Au delà de 80 MPa, des décollements de la roue 2 ne permettraient plus de traiter correctement les signaux.

Le moment d'excentricité des balourds est de 0,3 mkg pour une faible amplitude de vibration et de 0,6 mkg pour une grande amplitude.

Les conditions d'utilisation de l'appareil 1 sont les conditions habituelles des essais de mesure de portance des plateformes, c'est-à-dire que la plateforme 10 ne doit pas comporter de partie gelée.

L'appareil 1 est capable d'effectuer des mesures en pente longitudinale jusqu'à 7% et en pente transversale jusqu'à 5%. En dehors de ces conditions, les mesures effectuées peuvent présenter un léger biais.

Il est inutile d'effectuer sur la plateforme 10, un pré-passage d'un matériel quelconque. Le respect des conditions de non-glissance des plateformes 10 admises par les règles de l'art satisfait normalement à la traficabilité de l'ensemble véhicule 9, plus remorque 4 dans le domaine cité précédemment

**Revendications**

1. Procédé pour mesurer en continu la raideur d'une plateforme (10) d'infrastructure de transport et sols industriels, dans lequel

   - on fait rouler sur la plateforme (10) une roue (2) de masse Mr suspendue sous un châssis (3) de masse Mc, et
   - on soumet la roue (2) à des vibrations verticales au moyen d'un balourd (13) tournant autour de l'axe (6) de la roue (2) et soumis à une force centrifuge Fc,

**caractérisé par** les étapes suivantes :

- on mesure en fonction du temps l'accélération verticale Gr de la roue (2) et l'accélération verticale Gc du châssis (3) au moyen de capteurs d'accélération (14, 15) montés sur la roue (2) et sur le châssis (3),
- on mesure en fonction du temps la phase angulaire $\varphi$ entre la direction de la force centrifuge Fc du balourd et la verticale au moyen d'un capteur (16),
- on calcule en fonction du temps la composante verticale de la force appliquée FTA par la roue (2) sur la plateforme (10),
- on calcule en fonction du temps la déformation d verticale subie par la plateforme (10) à partir de la mesure de l'accélération verticale Gr de la roue (2),
- on établit pour chaque cycle de vibration la boucle (bl) de la composante verticale (FTA) par rapport à la déformation d,
- on calcule à chaque cycle de vibration la pente de la boucle dans la branche (b2) des composantes verticales ascendantes pour obtenir la raideur de la plate-forme (10).

2. Procédé selon la revendication 1, **caractérisé par le fait que** l'on calcule la pente de la boucle dans la plage des composantes verticales ascendantes comprises entre 30% et 90% de la composante verticale maximale (Fm).

3. Procédé selon l'une quelconque des revendications 1 ou 2, **caractérisé par le fait que** l'on calcule le module élastique de la plateforme en multipliant la valeur de la raideur par un coefficient d'étalonnage C déterminé par une expérimentation préalable.

4. Procédé selon l'une quelconque des revendications 1 à 3, **caractérisé par le fait que** la fréquence des vibrations est comprise entre 20 et 50 Hz.

5. Procédé selon l'une quelconque des revendications 1 à 4, **caractérisé par le fait que** l'on fait avancer la roue (2) à une vitesse voisine de 1m/s.

6. Procédé selon l'une quelconque des revendications 1 à 5, **caractérisé par le fait que** l'on mesure en continu la vitesse de la roue (2).

7. Procédé selon l'une quelconque des revendications 1 à 6, **caractérisé par le fait que** l'on calcule la raideur moyenne de la plateforme par unité de parcours en calculant la moyenne des raideurs calculées pour les cycles de vibration correspondant à cette unité de parcours,

8. Procédé selon la revendication 7, **caractérisé par le fait que** l'on affiche en continu les raideurs moyennes calculées en fonction de la distance sur un écran de visualisation.

9. Un appareil pour mettre en oeuvre le procédé selon l'une quelconque des revendications 1 à 8, **caractérisé par le fait qu'**il comporte :

- une remorque (4) tractable reposant sur des roues de roulement (7),
- un châssis (3) de masse Mc monté sur la remorque (4) de manière à pouvoir pivoter autour d'un axe transversal (5),
- une roue (2) de masse Mr équipée d'un balourd (13) et suspendue sous le châssis (3),
- des moyens pour faire tourner le balourd (13),
- un premier capteur d'accélération (15) monté sur le châssis (3),
- un deuxième capteur d'accélération (14) monté sur la roue (2),
- un capteur (16) d'angle du balourd (13),
- des moyens pour calculer en fonction du temps la déformation verticale (d) subie par la plateforme,
- des moyens pour établir à chaque cycle de vibration la boucle de la composante verticale (FTA) par rapport à la déformation (d), et des moyens pour calculer la raideur de la plateforme (10) à chaque cycle de vibration.

## Patentansprüche

1. Verfahren zur kontinuierlichen Messung der Tragfähigkeit eines Unterbaus (10) für den Transport oder von Industrieböden, wobei

- man über den Unterbau (10) ein Rad (2) mit der Masse Mr rollt, das an einem Rahmen (3) mit der Masse Mc aufgehängt ist, und
- man das Rad (2) mittels einer Unwucht (13), die sich um die Achse (6) des Rades (2) dreht und einer Zentrifugalkraft Fc ausgesetzt wird, Vertikalschwingungen unterwirft,

**gekennzeichnet durch** folgende Verfahrensschritte:

- man misst in Abhängigkeit von der Zeit die Vertikalbeschleunigung Gr des Rades (2) und der Vertikalbeschleunigung Gc des Rahmens (3) mittels am Rad (2) und am Rahmen (3) montierter Beschleunigungsmessfühler (14, 15),
- man misst in Abhängigkeit von der Zeit den Phasenwinkel (φ) zwischen der Richtung der Zentrifugalkraft Fc der Unwucht und der Vertikalen mittels eines Messfühlers (16),
- man berechnet in Abhängigkeit von der Zeit die Vertikalkomponente der **durch** das Rad (2) auf den Unterbau (10) aufgebrachten Kraft FTA,
- man berechnet in Abhängigkeit von der Zeit die Vertikaldeformation des Unterbaus (10) von der Messung der Vertikalbeschleunigung Gr des Rades (2) an,
- man erstellt für jeden Vibrationszyklus die Schleifenkurve (b1) der Vertikalkomponente (FTA) bezogen auf die Deformation d,
- man berechnet für jeden Vibrationszyklus die Neigung der Schleifenkurve am Ast (b2) der ansteigenden Vertikalkomponenten, um die Tragfähigkeit des Unterbodens (10) zu erhalten.

2. Verfahren nach Anspruch 1, **dadurch gekennzeichnet, dass** man die Neigung der Schleifenkurve im Bereich der ansteigenden Vertikalkomponenten berechnet, die zwischen 30 % und 90 % der maximalen Vertikalkomponente (Fm) sind.

3. Verfahren nach einem der Ansprüche 1 oder 2, **dadurch gekennzeichnet, dass** man den Elastizitätsmodul des Unterbaus durch Multiplizieren des Tragfähigkeitswertes mit einem Eichkoeffizienten C, der vorher experimentell bestimmt wurde, berechnet.

4. Verfahren nach einem der Ansprüche 1 bis 3, **dadurch gekennzeichnet, dass** die Vibrationsfrequenz zwischen 20 und 50 Hz ist

5. Verfahren nach einem der Ansprüche 1 bis 4, **dadurch gekennzeichnet, dass** man das Rad (2) mit einer Geschwindigkeit von etwa 1 m/s vorwärts bewegt.

6. Verfahren nach einem der Ansprüche 1 bis 5, **dadurch gekennzeichnet, dass** man kontinuierlich die Geschwindigkeit des Rades (2) misst.

7. Verfahren nach einem der Ansprüche 1 bis 6, **dadurch gekennzeichnet, dass** man die durchschnittliche Tragfähigkeit des Unterbaus pro Streckeneinheit berechnet, indem das Mittel der Tragfähigkeit für die Vibrationszyklen berechnet wird, das jener für eine Streckeneinheit errechneten entspricht.

8. Verfahren nach Anspruch 7, **dadurch gekennzeichnet, dass** man kontinuierlich die in Abhängigkeit von der Wegstrecke errechneten Tragfähigkeitsmittel auf einem Bildschirm anzeigt.

9. Vorrichtung zur Durchführung des Verfahrens nach einem der Ansprüche 1 bis 8 **dadurch gekennzeichnet, dass** sie umfasst:

- einen auf Laufrädern (7) aufruhenden Schlepperanhänger (4),
- einen Rahmen (3) mit der Masse Mc, der auf dem Schlepperanhänger (4) um eine Querachse (5) verschwenkbar montiert ist,
- ein Rad (2) mit der Masse Mr, das mit einer Unwucht (13) versehen und am Rahmen (3) aufgehängt ist,
- Mittel für den Drehantrieb der Unwucht (13),
- einen ersten auf dem Rahmen (3) angeordneten Beschleunigungsmessfühler (15),
- einen zweiten auf dem Rad (2) angeordneten Beschleunigungsmessfühler (14),
- einen Messfühler (16) für den Winkel der Unwucht (13),
- Mittel zur Berechnung der Vertikaldeformation (d) des Unterbaus (10) in Abhängigkeit von der Zeit,
- Mittel zur Erstellung der Schleifenkurve der Vertikalkomponente (FTA) bezogen auf die Deformation d für jeden Vibrationszyklus und Mittel zur Berechnung der Tragfähigkeit des Unterbaus (10) für jeden Vibrationszyklus.

**Claims**

1. A method for continuously measuring the stiffness of a transport infrastructure road level (10) and industrial floors, in which

   - a wheel (2) of mass Mr suspended beneath a chassis (3) of mass Mc is rolled on the road level (10),
   - the wheel (2) is subjected to vertical vibrations by means of an unbalanced member (13) rotating about the axis (6) of the wheel (2) and subjected to a centrifugal force Fc,

   the method being **characterised by** the following steps:

   - measuring as a function of time the vertical acceleration Gr of the wheel (2) and the vertical acceleration Gc of the chassis (3) by means of acceleration sensors (14, 15) mounted on the wheel (2) and on the chassis (3),
   - measuring as a function of time the angular phase φ between the direction of the centrifugal force Fc of the unbalanced member and the vertical by means of a sensor (16),
   - calculating as a function of time the vertical component FTA of the force applied by the wheel (2) on the road level (10),
   - calculating as a function of time the vertical deformation d undergone by the road level (10) from the measurement of the vertical acceleration Gr of the wheel (2),
   - establishing for each cycle of vibration the loop (b1) of the vertical component (FTA) with respect to the deformation d,
   - calculating at each cycle of vibration the gradient of the loop in the branch (b2) of the ascending vertical components in order to obtain the stiffness of the road level (10).

2. The method according to Claim 1, **characterised by** the fact that the gradient of the loop is calculated in the range of ascending vertical components included between 30% and 90% of the maximum vertical component (Fm).

3. The method according to Claim 1 or 2, **characterised by** the fact that the elastic modulus of the road level is calculated by multiplying the value of the stiffness by a coefficient of calibration C determined by prior experimentation.

4. The method according to any one of Claims 1 to 3, **characterised by** the fact that the frequency of the vibrations is included between 20 and 50 HZ.

5. The method according to any one of Claims 1 to 4, **characterised by** the fact that the wheel (2) is advanced at a speed close to 1 m/sec.

6. The method according to any one of Claims 1 to 5, **characterised by** the fact that the speed of the wheel (2) is measured continuously.

7. The method according to any one of Claims 1 to 6, **characterised by** the fact that the average stiffness of the road level per unit of distance covered is calculated by calculating the average of the stiffnesses calculated for the cycles of vibrations corresponding to this unit of distance covered.

8. The method according to Claim 7, **characterised by** the fact that the average stiffnesses calculated as a function of the distance are continuously displayed on a display screen.

9. An apparatus for carrying out the method according to any one of Claims 1 to 8, **characterised by** the fact that it comprises:

   - a towable trailer (4) resting on wheels (7) for rolling over the ground,
   - a chassis (3) of mass Mc mounted on the trailer (4) so as to be able to pivot about a transverse axle (5),
   - a wheel (2) of mass Mr equipped with an unbalanced member (13) and suspended beneath the chassis (3),
   - means for rotating the unbalanced member (13),
   - a first acceleration sensor (15) mounted on the chassis (3),

- a second acceleration sensor (14) mounted on the wheel (2),
- a sensor (16) detecting the angle of the unbalanced member (13),
- means for calculating the vertical deformation (d) undergone by the road level, as a function of time,
- means for establishing, upon each cycle of vibration, the loop of the vertical component (FTA) with respect to the deformation (d), and means for calculating the stiffness of the road level (10) upon each cycle of vibration.

FIG.1

FIG.3

FIG.2

FIG.4

FIG.5

Mc

3

V

2

Fc

Mr

V

φ

10

FTA

FIG.9

14

15

19

16

18

20

17

FIG.6

FIG.7

FIG.8

13